(19) Europäisches Patentamt
European Patent Office
Office européen des brevets

(11) Veröffentlichungsnummer: **0 415 884 A1**

(12) **EUROPÄISCHE PATENTANMELDUNG**

(21) Anmeldenummer: **90810625.5**

(51) Int. Cl.5: **C07C 233/43**, C07C 231/12

(22) Anmeldetag: **20.08.90**

(30) Priorität: **28.08.89 CH 3105/89**

(43) Veröffentlichungstag der Anmeldung:
**06.03.91 Patentblatt 91/10**

(84) Benannte Vertragsstaaten:
**CH DE FR GB LI**

(71) Anmelder: **CIBA-GEIGY AG**
**Klybeckstrasse 141**
**CH-4002 Basel(CH)**

(72) Erfinder: **Palluat de Besset, Amaury**
**Rue de la Lisière 2**
**F-68100 Mulhouse(FR)**

(54) **Verfahren zur Herstellung von aromatischen Aminen.**

(57) Die Erfindung betrifft ein Verfahren zur Herstellung von Verbindungen der Formel

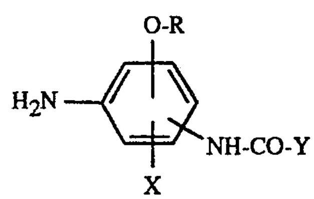

(1),

worin R gegebenenfalls substituiertes $C_1$-$C_4$-Alkyl oder gegebenenfalls substituiertes Phenyl, X Wasserstoff oder gegebenenfalls substituiertes $C_1$-$C_4$-Alkyl, und Y gegebenenfalls substituiertes $C_1$-$C_4$-Alkyl ist, welches dadurch gekennzeichnet ist, dass man Verbindungen der Formel

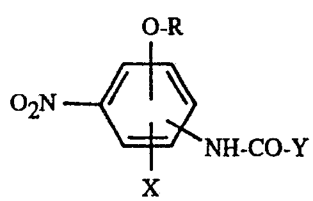

(2),

worin R, X und Y die unter Formel (1) angegebenen Bedeutungen haben, bei alkalischem pH-Wert katalytisch hydriert.

Die nach dem erfindungsgemässen Verfahren erhaltenen Verbindungen eignen sich als Zwischenprodukte zur Herstellung von Farbstoffen.

EP 0 415 884 A1

# VERFAHREN ZUR HERSTELLUNG VON AROMATISCHEN AMINEN

Die Erfindung betrifft ein neues Verfahren zu Herstellung von aromatischen Aminen, die einen Alkanoylaminorest und einen Alkoxy- oder Phenoxyrest enthalten, aus den entsprechenden aromatischen Nitroverbindungen durch katalytische Hydrierung bei alkalischem pH-Wert.

In den letzten Jahren ist man zunehmend bestrebt, Herstellungsverfahren für chemische Verbindungen, insbesondere für Farbstoffe und deren Zwischenprodukte, zu optimieren, und zwar sowohl was den Herstellungsprozess anbetrifft, als auch hinsichtlich der Aufarbeitung. Um hier zu befriedigenden und reproduzierbaren Ergebnissen zu gelangen, ist man auf Herstellungsverfahren angewiesen, die sich durch folgende Kriterien auszeichnen: vollständige Umsetzung, geringer Nebenproduktanteil, einfache Verfahrensführung, Automationsfähigkeit, kurze Verweilzeiten in den Reaktionsgefässen und einfachste Aufarbeitungs- bzw. Abtrennmethode.

Zur Herstellung von aromatischen Aminen aus den entsprechenden Nitroverbindungen sind aus der Literatur zahlreiche Verfahren bekannt. Nicht bekannt sind Herstellungsverfahren von aromatischen Aminen aus den entsprechenden Nitroverbindungen, die einen Alkanoylaminorest und einen Alkoxy- oder Phenoxyrest gebunden enthalten. Nachteil der meisten dieser Reduktionen bzw. Hydrierungsreaktionen ist sowohl die unbefriedigende Ausbeute als auch der hohe Anteil an Nebenprodukten.

Sowohl für kontinuierliche aus auch für diskontinuierliche Herstellungsverfahren für Farbstoffe und deren Zwischenprodukte wird heute unabhängig von der Quälitat der Ausgangsmaterialien eine gleichbleibende Qualität der Verfahrensprodukte bei optimaler Ausbeute verlangt.

Insbesondere diskontinuierliche Herstellungsverfahren (sog. batch-Prozesse) führen leicht zu schwankenden Qualitäten der Verfahrensprodukte bedingt durch unterschiedliche Qualität der Ausgangsverbindungen, und die Ausbeute ist von Partie zu Partie verschieden.

Ueberraschenderweise erlaubt das erfindungsgemässe Verfahren die Herstellung von aromatischen Aminen, die einen Alkanoylaminorest und einen Alkoxy- oder Phenoxyrest enthalten, aus den entsprechenden Nitroverbindungen auf einfache Art und Weise, ohne die genannten Nachteile aufzuweisen, insbesondere wird gemäss dem erfindungsgemässen Verfahren der Anteil der Nebenprodukte stark vermindert.

Gegenstand der Erfindung ist ein Verfahren zur Herstellung von Verbindungen der Formel

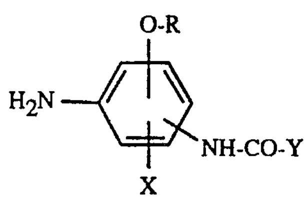 (1),

worin R gegebenenfalls substituiertes $C_1$-$C_4$-Alkyl oder gegebenenfalls substituiertes Phenyl, X Wasserstoff oder gegebenenfalls substituiertes $C_1$-$C_4$-Alkyl, und Y gegebenenfalls substituiertes $C_1$-$C_4$-Alkyl ist, welches dadurch gekennzeichnet ist, dass man Verbindungen der Formel

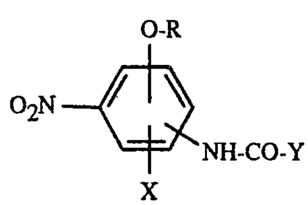 (2),

worin R, X und Y die unter Formel (1) angegebenen Bedeutungen haben, bei alkalischem pH-Wert katalytisch hydriert.

Bei diesem Vorgehen erhält man überraschenderweise Amine der Formel (1) in nahezu quantitativer Ausbeute mit einem sehr geringen Nebenproduktanteil.

Als $C_1$-$C_4$-Alkyl kommt für die Reste R, X und Y, unabhängig voneinander in Betracht: Methyl, Aethyl, Isopropyl, n-Propyl, Isobutyl, n-Butyl, sec-Butyl und tert. Butyl, wobei die genannten Reste weitersubstituiert sein können, wie z.B. durch Hydroxy, Halogen, wie Fluor, Chlor oder Brom, $C_1$-$C_4$-Alkoxy, wie Methoxy,

Aethoxy, Isopropoxy oder n-Butoxy, Cyan, Carboxy, Carbamoyl, $C_1$-$C_4$-Alkoxycarbonyl, wie z.B. Methoxycarbonyl und Aethoxycarbonyl, $C_1$-$C_4$-Alkylcarbonyloxy, wie z.B. Acetyloxy, Sulfo und Sulfamoyl. Als Beispiele für derartig substituierte Reste R, X und Y seien genannt:

2-Hydroxyäthyl, 2-Hydroxvpropyl, 3-Hydroxypropyl, 2-Hydroxybutyl, 3-Hydroxybutyl, 4-Hydroxybutyl, 2,3-Dihydroxypropyl, 2,4-Dihydroxyburyl, Cyanmethyl, 2-Cyanäthyl, 3-Cyanpropyl, Methoxymethyl, Aethoxymethyl, 2-Methoxyäthyl, 2-Aethoxyäthyl, 3-Methoxypropyl, 3-Aethoxypropyl, 2-Hydroxy-3-methoxypropyl, Chlormethyl, 2-Chloräthyl, 2-Bromäthyl, Brommethyl, 3-Chlorpropyl, 4-Chlorbutyl, Carboxymethyl, 2-Carboxyäthyl, 3-Carboxypropyl, 4-Carboxybutyl, 1 ,2-Dicarboxyäthyl, Carbamoylmethyl, 2-Carbamoyläthyl, 3-Carbamoylpropyl, Methoxycarbonylmethyl, 2-Methoxycarbonyläthyl, 4-Methoxycarbonylbutyl, 4-Aethoxycarbonylbutyl, Methylcarbonyloxymethyl, 3-Methylcarbonyloxypropyl, 3-Aethylcarbonyloxypropyl, 4-Methylcarbonyloxybutyl, Sulfomethyl, 2-Sulfoäthyl, 2-Sulfopropyl, Sulfamoylmethyl, 2-Sulfamoyläthyl.

Als Phenyl kommt für R ein unsubstituierter oder substituierter Phenylrest in Betracht.

Als Substituenten des Phenylrestes kommen z.B. die folgenden in Betracht: $C_1$-$C_4$-Alkyl, wie Methyl, Aethyl, Propyl, Isopropyl, Butyl, Isobutyl, sek.-Butyl und tert.-Buryl, $C_1$-$C_4$-Alkoxy, wie Methoxy, Aethoxy, Propoxy, Isopropoxy, sek.-Butoxy, Isobutoxy und n-Butoxy, Halogen, wie Fluor, Chlor und Brom und Sulfo. Bevorzugt sind die Substituenten Methyl, Aethyl, Methoxy, Aethoxy, Chlor, Brom und Sulfo.

In dem erfindungsgemässen Verfahren werden vorzugsweise Verbindungen der Formel (2) verwendet, worin der Rest R und/oder der Rest X und/oder der Rest Y als $C_1$-$C_4$-Alkyl nicht weitersubstituiert ist. Ebenfalls bevorzugt werden Verbindungen der Formel (2) in dem erfindungsgemässen Verfahren verwendet, worin R als Phenylrest nicht weitersubstituiert ist.

Eine besonders bevorzugte Ausführungsform des erfindungsgemässen Verfahrens ist dadurch gekennzeichnet, dass man bei einem pH-Wert von 7,5 bis 11, insbesondere von 8 bis 10, hydriert. Der pH-Wert wird alkalisch gestellt durch Zugabe von alkalisch wirkenden Mitteln, insbesondere hat sich Ammoniak bzw. wässrige Ammoniak-Lösung mit 20 bis 40 Gewichtsprozent Ammoniak als vorteilhaft erwiesen. In dem erfindungsgemässen Verfahren erfolgt die Zugabe des alkalisch wirkenden Mittels, insbesondere des Ammoniaks vorzugsweise in der Menge, die ausreicht um den pH-Wert einzustellen. Im Fall von Ammoniak hat sich eine Menge von 4 bis 40 Molprozent, insbesondere von 4 bis 12 Molprozent, bezogen auf die Molmenge der Verbindung der Formel (2), als günstig erwiesen.

Das erfindungsgemässe Verfahren wird in Gegenwart von Hydrierungskatalysatoren ausgeführt. Als Hydrierungskatalysatoren kommen insbesondere Edelmetallkatalysatoren, wie z.B. Platin-, Palladium-, Nickel- und Kupferchromit-Katalysatoren in Betracht. Die erforderliche Reaktionstemperatur hängt im wesentlichen vom verwendeten Katalysator ab. Reaktionstemperaturen zwischen 30 und 250 °C haben sich als günstig erwiesen, vorzugsweise erfolgt die Umsetzung bei einer Temperatur zwischen 30 und 160 °C, insbesondere zwischen 50 und 120 °C, wobei sich als Hydrierungskatalysator Palladium/Aktivkohle als besonders geeignet erwiesen hat. Die Umsetzung kann unter Atmosphärendruck oder mit Ueberdruck erfolgen. Falls die Umsetzung mit Ueberdruck ausgeführt wird, liegt der Ueberdruck im allgemeinen zwischen 0,1 und 100 bar, insbesondere zwischen 1 und 30 bar. Bevorzugt erfolgt die Umsetzung unter Ueberdruck.

Die Hydrierung kann z.B. in Substanz oder in einem inerten Lösungsmittel durchgeführt werden. Geeignete Lösungsmittel sind z.B. Alkanole (Methanol, Aethanol, Propanol, Butanol, Methoxy- oder Aethoxyäthanol), Aether (Dibutyläther, t-Butylmethyläther Tetrahydrofuran, Dioxan, Aethylenglykoldimethyläther, Diäthylenglykoldimethyläther), Amide und Lactame (Dimethylformamid, Dimethylacetamid, N-Methylpyrrolidon), Ester und Lactone (Essigsäureäthylester, G-Butyrolacton), Kohlenwasserstoffe (Pentan, Hexan, Methylcyclohexan, Benzol, Toluol, Xylol, Chlorbenzol), Wasser. Bei der Hydrierung in Substanz ist das bei der Hydrierung entstehende Amin das Lösungsmittel.

In einer bevorzugten Ausführungsform wird als Lösungsmittel ein $C_1$-$C_4$-Alkanol alleine oder im Gemisch mit Wasser verwendet. Insbesondere wird Methanol verwendet. Das Lösungsmittel wird in der 2- bis 100-fachen Molmenge der Verbindung der Formel (2) eingesetzt.

Die Reaktionstemperatur beträgt vorteilhaft 50 bis 120 °C. Der Druck beträgt vorzugsweise 1 bis 30 bar. Die Reaktionszeit richtet sich im wesentlichen nach den Reaktionsbedingungen und beträgt im allgemeinen weniger als zwei Stunden.

Das erfindungsgemässe Verfahren kann so durchgeführt werden, dass man in einem Autoklaven die Nitroverbindung, den Hydrierungskatalysator, das Lösungsmittel und Ammoniak einträgt und die Luft zuerst durch Stickstoff und diesen dann durch Wasserstoff verdrängt. Dann wird der Autoklav verschlossen, Wasserstoff bis zum gewünschten Druck aufgepresst und auf die Reaktionstemperatur erhitzt. Nach Beendigung der Reaktion wird das Reaktionsgemisch vom Katalysator abgetrennt. Danach kann man das Reaktionsprodukt vom Reaktionswasser und Lösungsmittel abtrennen und durch Destillation oder Umkristallisation weiter reinigen. Die Amine der Formel (1) sind bekannterweise Zwischenprodukte zur Herstellung

von Farbstoffen.

Ganz besonders bevorzugte Ausführungsformen des erfindungsgemässen Verfahrens sind dadurch gekennzeichnet, dass man Verbindungen der Formel (2) verwendet, worin - X Wasserstoff ist; - R Methyl, Aethyl oder Phenyl, vorzugsweise Methyl; und - Y Methyl oder Aethyl ist; oder worin - X Wasserstoff, R Methyl und Y Methyl oder Aethyl ist.

Eine wichtige Ausführungsform des erfindungsgemässen Verfahrens ist dadurch gekennzeichnet, dass man Verbindungen der Formel

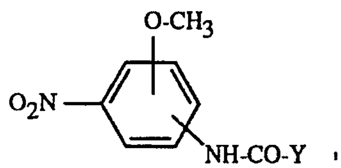

(3),

worin Y′ Methyl oder Aethyl ist, in Methanol in Gegenwart von Ammoniak bei einem pH-Wert von 8,5 bis 10 in Gegenwart von Palladium/Aktivkohle hydriert; vorzugsweise wird die Verbindung der Formel (3) in Gegenwart von 4 bis 12 Molprozent Ammoniak, bezogen auf die Molmenge der Verbindung der Formel (3) hydriert, wobei bezogen auf das Gewicht der Verbindung der Formel (3) in Gegenwart von 0,2 bis 1,0, insbesondere in Gegenwart von 0,3 bis 0,5 Gewichtsprozent Palladium(Aktivkohle hydriert wird.

Eine besonders wichtige Ausführungsform des erfindungsgemässen Verfahrens ist dadurch gekennzeichnet, dass man die Verbindung der Formel

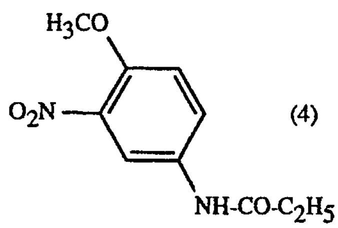

(4)

in Methanol in Gegenwart von 4 bis 12 Molprozent Ammoniak bezogen auf die Molmenge der Verbindung der Formel (4) und in Gegenwart von 0;3 bis 0,5 Gewichtsprozent Palladium/Aktivkohle bezogen auf das Gewicht der Verbindung der Formel (4) bei einem Druck von 15 bis 25 bar zu der Verbindung der Formel

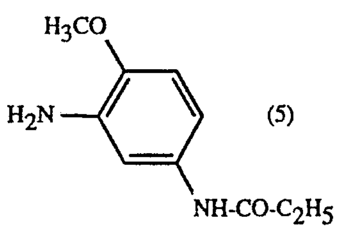

(5)

hydriert.

Als Ausgangsverbindungen der Formel (2) sei beispielsweise genannt: 2-Nitro-4-propionamidoanisol. 2-Nitro-5-acetamidoanisol, 2-Nitro-4- oder -5-proprionamido- oder -4- oder -5-acetamidophenetol, 1-Nitro-2-phenoxy-5-propionamidobenzol.

Die Verbindungen der Formel (2) sind an sich bekannt oder können in Analogie zu bekannten Verbindungen erhalten werden.

Das erfindungsgemässe Verfahren weist gegenüber den bekannten Verfahren zur Herstellung der Amine der Formel (1) folgende Vorteile auf:

Durch die selektive Reduktion bzw. Hydrierung der aromatischen Nitroverbindung der Formel (2) in Gegenwart eines alkalisch wirkenden Mittels, insbesondere Ammoniak und in Gegenwart von Hydrierungskatalysatoren, insbesondere PalladiumAktivkohle kann die Umsetzung quantitativer erfolgen als es bisher möglich war.

Durch die höhere Reinheit der gemäss dem erfindungsgemässen Verfahren erhaltenen Verbindungen verglichen mit den auf übliche Weise hergestellt gleichen Verbindungen können Farbstoffe mit reproduzierbar verbesserten Eigenschaften erhalten werden.

Das nachfolgende Beispiel dient der Veranschaulichung der Erfindung. Darin sind die Teile Gewichtsteile und die Prozente Gewichtsprozente. Die Temperaturen sind in Celsiusgraden angegeben. Die Beziehung zwischen Gewichtsteilen und Volumenteilen ist dieselbe wie diejenige zwischen Gramm und Kubikzentimeter.

Beispiel 1 : 114 Teile 2-Nitro-4-propionamidoanisol, 160 Teile Methanol, 2,5 Teile 30%ige wässrige Ammoniaklösung und 1 Teil einer 50%igen wasserfeuchten Palladium/Aktivkohle-Suspension in 2 Teilen Wasser werden in einem Autoklaven mit Begasungsrührer eingetragen und Wasserstoff aufgepresst. Bei einem Druck von 18 bar und einer Temperatur von 80° wird 1 Stunde hydriert. Man erhält in quantitativer Ausbeute 2-Methoxy-5-propionamidoanilin mit einer Reinheit von >97 % (flüssigchromatophische Analyse).

Beispiel 2 : 104 Teile 2-Nitro-4-acetamidoanisol, 160 Teile Aethanol, 12,5 Teile 30%ige wässrige Ammoniaklösung und 1 Teil einer 50%igen wasserfeuchten Palladium/Aktivkohle-Suspension in 2 Teilen Wasser werden in einem Autoklaven mit Begasungsrührer eingetragen und Wasserstoff aufgepresst. Bei einem Druck von 8 bar und einer Temperatur von 100° wird 2 Stunden hydriert. Man erhält in quantitativer Ausbeute 2-Methoxy-5-acetamidoanilin mit einer Reinheit von >97% (flüssigchromatophische Analyse).

Beispiel 3 :114 Teile 2-Nitro-4-propionamidoanisol, 160 Teile Propanol, 3,4 Teile 30%ige wässrige Ammoniaklösung und 2 Teile einer 50%igen wasserfeuchten Palladium/Aktivkohle-Suspension in 3 Teilen Wasser werden in einem Autoklaven mit Begasungsrührer eingetragen und Wasserstoff aufgepresst. Bei einem Druck von 25 bar und einer Temperatur von 120° wird 1 Stunden hydriert. Man erhält in quantitativer Ausbeute 2-Methoxy-5-propionamidoanilin mit einer Reinheit von > 97% (flüssigchromatophische Analyse.

Beispiel 4 :104 Teile 2-Nitro-4-acetamidoanisol, 200 Teile Methanol, 1,2 Teile 30%ige wässrige Ammoniaklösung und 2 Teile einer 50%igen wasserfeuchten Palladium/Aktivkohle-Suspension in 2 Teilen Wasser werden in einem Autoklaven mit Begasungsrührer eingetragen und Wasserstoff aufgepresst. Bei einem Druck von 5 bar und einer Temperatur von 100° wird 2 Stunden hydriert. Man erhält in quantitativer Ausbeute 2-Methoxy5-acetamidoanilin mit einer Reinheit von >97% (flüssigchromatophische Analyse).

Wenn man wie in den Beispielen 1 bis 4 angegeben verfährt und anstelle der in den Beispielen 2 bis 4 angegebenen Nitoverbindung eine äquimolare Menge einer in Spalte 2 der nachfolgenden Tabelle angegebenen Nitroverbindung verwendet, so erhält man eine der in Spalte 3 der nachfolgenden Tabelle angegebenen Aminoverbindungen.

Tabelle

| Beispiel | Nitroverbindung | Aminoverbindung |
|---|---|---|
| | 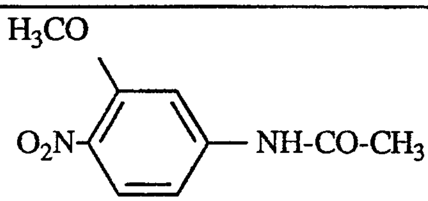 | 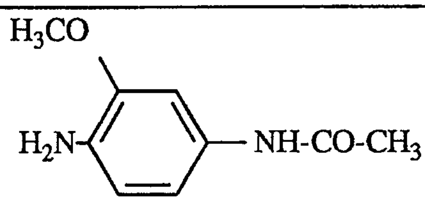 |
| | 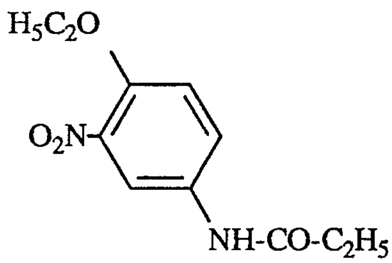 | 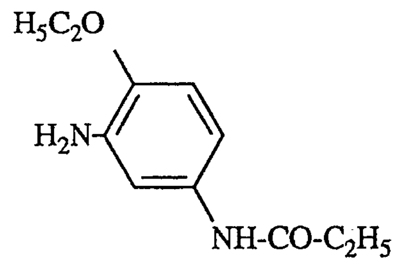 |
| | 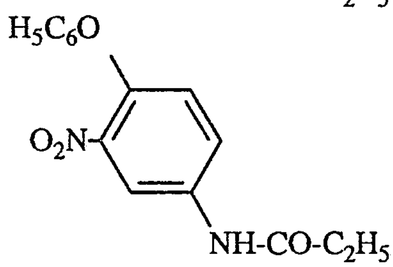 | 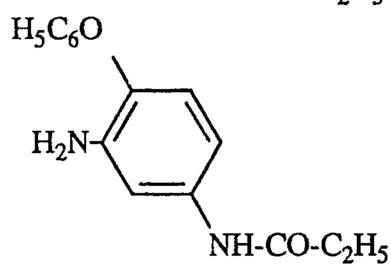 |
| | 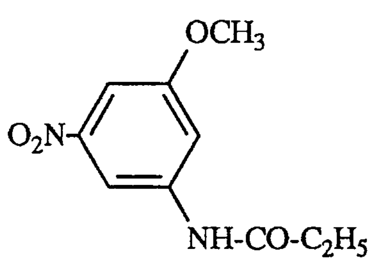 | 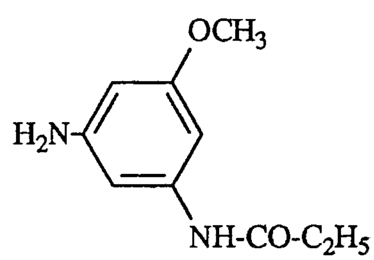 |
| | 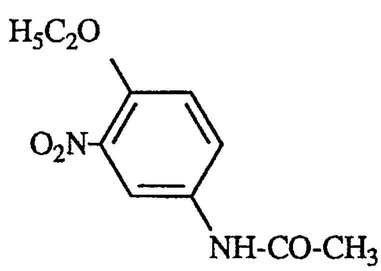 | 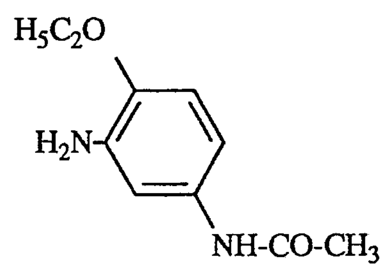 |
| | 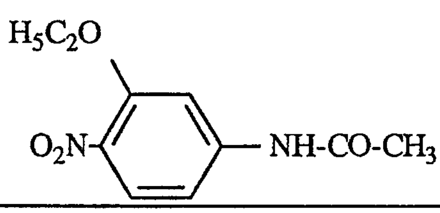 | 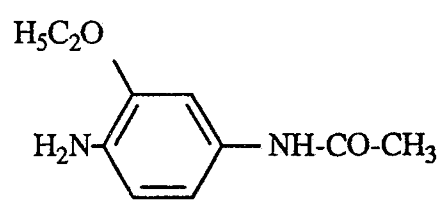 |

## Ansprüche

1. Verfahren zur Herstelung von Verbindungen der Formel

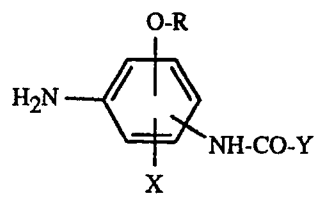

(1),

worin R gegebenenfalls substituiertes $C_1$-$C_4$-Alkyl oder gegebenenfalls substituiertes Phenyl, X Wasserstoff oder gegebenenfalls substituiertes $C_1$-$C_4$-Alkyl, und Y gegebenenfalls substituiertes $C_1$-$C_4$-Alkyl ist, dadurch gekennzeichnet, dass man Verbindungen der Formel

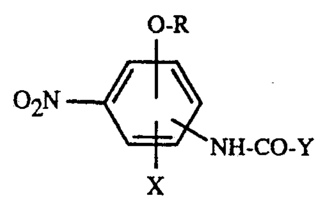

(2),

worin R, X und Y die unter Formel (1) angegebenen Bedeutungen haben, bei alkalischem pH-Wert katalytisch hydriert.

2. Verfahren gemäss Anspruch 1, dadurch gekennzeichnet, dass man bei einem pH-Wert zwischen 7,5 und 11, insbesondere zwischen 8 und 10, hydriert.

3. Verfahren gemäss einem der Ansprüche 1 und 2, dadurch gekennzeichnet, dass man in einem organischen Lösungsmittel hydriert.

4. Verfahren gemäss einem der Ansprüche 1 bis 3, dadurch gekennzeichnet, dass man bei einem Druck von 0,1 bis 100 bar, insbesondere bei einem Druck von 1 bis 30 bar, hydriert.

5. Verfahren gemäss einem der Ansprüche 1 bis 4, dadurch gekennzeichnet, dass man bei einer Temperatur zwischen 30 und 250 °C, insbesondere zwischen 50 und 120 °C hydriert.

6. Verfahren gemäss einem der Ansprüche 1 bis 5, dadurch gekennzeichnet, dass man in Gegenwart von Palladium/Aktivkohle hydriert.

7. Verfahren gemäss einem der Ansprüche 1 bis 6, dadurch gekennzeichnet, dass man den alkalischen pH-Wert mit Ammoniak oder einer wässrigen Ammoniak-Lösung einstellt.

8. Verfahren gemäss einem der Ansprüche 1 bis 7, dadurch gekennzeichnet, dass man Verbindungen der Formel (2) hydriert, worin X Wasserstoff ist.

9. Verfahren gemäss einem der Ansprüche 1 bis 8, dadurch gekennzeichnet, dass man Verbindungen der Formel (2) hydriert, worin R Methyl, Aethyl oder Phenyl ist.

10. Verfahren gemäss Anspruch 9, dadurch gekennzeichnet, dass man Verbindungen der Formel (2) hydriert, worin R Methyl ist.

11. Verfahren gemäss einem der Ansprüche 1 bis 10, dadurch gekennzeichnet, dass man Verbindungen der Formel (2) hydriert, worin Y Methyl oder Aethyl ist.

12. Verfahren gemäss einem der Ansprüche 1 bis 11, dadurch gekennzeichnet, dass man Verbindungen der Formel

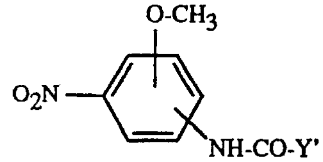

(3),

worin Y′ Methyl oder Aethyl ist, in Methanol in Gegenwart von Ammoniak bei einem pH-Wert von 8,5 bis 10 und in Gegenwart von Palladium Aktivkohle hydriert.

13. Verfahren gemäss Anspruch 12, dadurch gekennzeichnet, dass man in Gegenwart von 4 bis 12 Molprozent Ammoniak, bezogen auf die Molmenge der Verbindung der Formel (3), hydriert.

14. Verfahren gemäss einem der Ansprüche 12 und 13, dadurch gekennzeichnet, dass man bezogen auf das Gewicht der Verbindung der Formel (3) in Gegenwart von 0,2 bis 1,0, insbesondere in Gegenwart von 0,3 bis 0,5 Gewichtsprozent Palladium/Aktivkohle hydriert.

15. Verfahren gemäss einem der Ansprüche 12 bis 14, dadurch gekennzeichnet, dass man die Verbindung der Formel

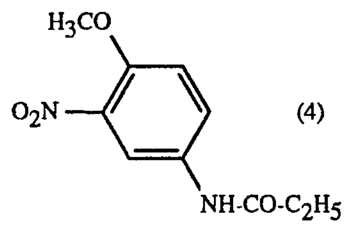

(4)

in Methanol in Gegenwart von 4 bis 12 Molprozent Ammoniak bezogen auf die Molmenge der Verbindung der Formel (4) und in Gegenwart von 0,3 bis 0,5 Gewichtsprozent Palladium/Aktivkohle bezogen auf das Gewicht der Verbindung der Formel (4) bei einem Druck von 15 bis 25 bar zu der Verbindung der Formel

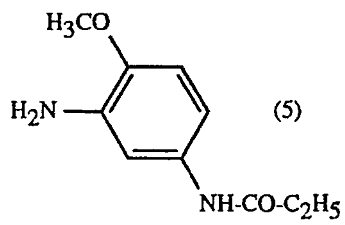

(5)

hydriert.

16. Die nach dem Verfahren gemäss Anspruch 1 erhaltenen Verbindungen der Formel (1).

17. Verwendung der gemäss dem Verfahren nach Anspruch 1 erhaltenen Verbindungen zur Herstellung von Farbstoffen.

Europäisches Patentamt

# EUROPÄISCHER RECHERCHENBERICHT

Nummer der Anmeldung

EP 90810625.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| Y | FR - A - 2 163 519 (CIBA-GEIGY) * Seite 8, Zeile 32 - Seite 9, Zeile 4 * -- | 1-6, 10,11 | C 07 C 233/43<br>C 07 C 231/12 |
| Y | EP - A2 - 0 165 564 (HOECHST) * Ansprüche; Seite 4, Zeile 23 - Seite 5, Zeile 3 * -- | 1-6, 10,11 | |
| A | US - A - 2 407 309 (LOTT et al.) * Spalte 8, Zeilen 64-70 * -- | 1,3,4, 10,11 | |
| A | CHEMICAL ABSTRACTS, Band 106, Nr. 6, 9. Februar 1987, Columbus, Ohio, USA A. KAZMERCZAK et al. "Catalytic liquid-phase reduction of 2-nitro-4-acetylaminoanisole" Seite 82, Zusammenfassung-Nr. 34 591r & Przem. Chem. 1986, 65(7), 358-60 ---- | 1,3-6, 10,11 | RECHERCHIERTE SACHGEBIETE (Int Cl 5)<br>C 07 C 233/00<br>C 07 C 231/00 |

Der vorliegende Recherchenbericht wurde für alle Patentansprüche erstellt.

| Recherchenort | Abschlußdatum der Recherche | Prüfer |
|---|---|---|
| WIEN | 03-12-1990 | KÖRBER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument